# EUROPEAN PATENT APPLICATION

(11) **EP 4 674 453 A1**
(43) Date of publication of application: **07.01.2026**
(21) Application number: 24186149.1
(22) Date of filing: 03.07.2024
(51) Int. Cl.: A61M 5/24

(54) **RESERVOIR CODING MECHANISM FOR DRUG DELIVERY DEVICE**

(71) Applicant: Ypsomed AG, 3401 Burgdorf (CH)
(72) Inventor: Bosshard, Simon Martin, 3324 Hindelbank (CH); Kalbermatter, Gabriel, 3400 Burgdorf (CH); Schrul, Christian, 3414 Oberburg (CH); Schindler, Sarah, 3018 Bern (CH); von Mühlenen, Beat, 3043 Uetligen (CH); Glauser, Oliver, 3008 Bern (CH)
(74) Representative: Kleiner, Stefan

(57) **Abstract**

The invention relates to a reservoir assembly (2) for a drug delivery device (1) for dispensing a liquid drug from a reservoir (3). The reservoir assembly comprises a reservoir holder (30) adapted to be connected to a dispensing mechanism of the delivery device and a coding collar (10) comprising fastening means for connecting the collar non-releasably to a neck portion of the reservoir (3). The collar (10) comprises a coding feature (10). The reservoir assembly (2) further comprises a coding ring (40) comprising a first connecting portion to connect non-releasably the coding ring to the distal end of the reservoir holder (30) and a second connecting portion to connect the coding ring (40) to the collar (10) with the reservoir (3).

## Description

### FIELD OF THE INVENTION

The present invention relates to a reservoir assembly for a drug delivery device for dispensing a liquid drug through an injection needle for injecting, delivering, administering, infusing or dispensing substances and/or liquids such as insulin, hormone preparations or vaccines. The reservoir assembly comprises a reservoir holder adapted to be connected to a dispensing mechanism of the delivery device and a coding collar comprising fastening means for connecting the collar to a neck portion of the reservoir.

### BACKGROUND OF THE INVENTION

A variety of diseases exist that require regular treatment by subcutaneous administration of a medicament, and a number of drug delivery devices have been developed to support a patient in accurately and controllably delivering an amount of drug in a self-administration process. Delivery devices include injection devices that are removed from the injection site after each medication event or drug delivery process, as well as infusion devices with a cannula or needle that remains in the skin of the patient for a prolonged period of time.

By way of example, diabetes may be treated by self-administration of insulin or its derivatives with the help of multi-variable-dose insulin injection pens. An injection pen device generally has an elongate device body defining a longitudinal main device axis. An automatic injection device has a motor or a drive spring for biasing a plunger rod and shifting a piston in a container barrel, wherein the drive spring may have to be charged or strained manually prior to injection of a dose. A manually powered delivery drive requires a user to manually provide the energy to move the piston, for instance by applying a distal force component to the injection device.

The medicament dose to be injected may be manually selected by turning a dosage knob and observing the actual dialed dose from a dose window or display of the injection pen. A dose is dispensed by inserting the needle into a suited portion of human skin and by moving the piston manually or by pressing a release button of an automatic injection device. Automatic injection devices may comprise an electronic dose dial mechanism to automatically set a dose.

The reusable or semi-reusable delivery device usually comprise a housing accommodating a dose and dispensing mechanism and a reservoir holder adapted to hold a reservoir, e.g. a cartridge or a syringe, in place relative to the housing. In order to prevent a user from connecting a wrong or inappropriate reservoir to the dose and dispensing mechanism, numerous reservoir coding and coupling systems have been developed.

By way of example, EP 2 091 600 B1 discloses a reusable injection pen comprising a housing and a cartridge holder releasably attachable to the housing by a bayonet connection. To ensure that only specific and predetermined cartridges are fastened to the housing, the cartridge holder defines coding projections which are adapted to be received in a corresponding coding groove of the housing.

WO 2022/233751 A1 discloses an injection pen with a housing accommodating a dose and dispensing mechanism. The housing includes longitudinal grooves to receive a correspondingly-shaped radially outwardly extending longitudinal projection on a sidewall of a cartridge holder. The longitudinal projections received in the longitudinal grooves provide a rotation inhibiting fastening and a respective sliding motion of the housing. The sliding axial insertion motion of the cartridge holder into the housing is delimited as an annular ridge axially abuts with a distal face of the sidewall of the housing.

EP 2 654 852 B1 discloses a coding system comprising a retention cap fixedly connected to a neck portion of a cartridge by a snap-fit connection between the retention cap and a ferrule of cartridge. The retention cap includes clips with a flange and a coding feature. When the cartridge is axially inserted into the cartridge holder from a distal end of the holder, the coding feature of the cap slides within a recess in the cartridge holder. If the coding feature is not compatible and does not match the shape of recess, the cartridge travel will be restricted, and cartridge will not be able to continue to be inserted within cartridge holder. If the coding feature does match with recess, cartridge is able to be fully inserted into cartridge holder and the latter can be subsequently connected to an injection pen housing.

### DESCRIPTION OF THE INVENTION

It is an objective of the invention to provide an easy customizable and adaptable coding mechanism allowing only a specific reservoir to be attached to a specific dose and dispensing mechanism of a drug delivery device.

This objective is achieved by a dose and dispensing mechanism according to the independent claims. Preferred embodiments are evident from the dependent claims.

The invention relates to a reservoir assembly for a drug delivery device for dispensing a liquid drug from a reservoir. The reservoir assembly comprises
- a reservoir holder defining a longitudinal axis and with a distal end and proximal end and adapted to be fixedly and non-releasably connected to a dispensing mechanism of the delivery device and wherein the reservoir holder is adapted to hold the reservoir in place relative to the dispensing mechanism;
- a coding collar comprising fastening means for connecting the collar fixedly and non-releasably to a neck portion of the reservoir, the collar comprising a coding feature.

The reservoir assembly further comprises a coding ring adapted to surround the reservoir, the coding ring comprising
- a first connecting portion to attach or to connect the coding ring fixedly and non-releasably to a distal end of the reservoir holder,
   a second connecting portion to releasably attach and couple the coding ring to the collar,
   wherein the second connection portion includes a counter coding feature adapted to exclusively match the coding feature of the collar. That means the second connection portion includes the counter coding feature which exclusively matches the coding feature such that the reservoir may be held in the reservoir holder if and only if the counter coding feature matches the coding feature. In the matching case the reservoir is releasably connectable and can be coupled to the reservoir holder via coding collar and coding ring by insertion of the reservoir into the reservoir holder.

The connection of the reservoir to the dose and dispensing mechanism by means of the coding collar and the coding ring prevents the user from connecting an inappropriate or wrong or non-compatible (disposable) reservoir with collar to a (reusable) housing of the dose and dispensing mechanism of delivery device. That means only a specific, correct and compatible reservoir with a corresponding collar can be attached to the housing of the dose and dispensing mechanism. The coding feature and the counter coding feature may be customized and configured to provide a definite (unique) coding for a particular collar with a reservoir, e.g. the coding may be unique for a specific drug or customer.

The reservoir must be connected to reservoir holder via collar (which is fixedly and non-releasably connected to the reservoir) and coding ring. Namely, only if the coding feature matches the counter coding feature the collar can be connected to the ring. For the attachment, a proximal portion of the reservoir has to be inserted into a distal opening of reservoir holder such that the reservoir is enclosed and enveloped by the reservoir holder and the coding ring which is fixedly connected to the reservoir holder.

The reservoir is non-releasably connected to the collar at a manufacturer site and not intended to be separated by the user. The term "non-releasably" means the collar cannot be disconnected and separated from the reservoir without destruction. Likewise, the coding ring is connected to the reservoir holder at a manufacturer side and not intended to be separated by the user.

The coding ring is a separate part and prior final assembly a specific ring with a specific or unique counter coding feature can be chosen by the manufacturer or distributor to allow only a specific collar and reservoir to be attached to dose and dispensing mechanism via coding ring by the user. As the ring is a separate part the coding can easily be adapted or customized. Unlike prior art approaches where the whole reservoir holder has to be replaced for a change or replace the coding according to the invention can be changed or replaced by only exchanging the coding ring. That means the volume and/or number of (plastic) parts to be replaced when changing the coding is reduced which is cost-effective and is advantageous from an environmental view.

The coding feature and the counter coding feature exclusively match to each other. That means if the coding feature does not match the counter coding feature the reservoir with the collar cannot be attached or connected to the coding ring by the user and thus cannot be connected to the reservoir holder. That means the coding feature and the counter coding feature provide an exclusive, releasable and reliable form-fit connection.

The coding feature and the counter coding feature may be, for example, a protrusion and a groove adapted to accommodate the protrusion or a rib, a cam, a knob or a lobe and a corresponding slot, recess, keyway or the coding feature may be a non-standard customized thread including a coding thread part and a counter coding thread part exclusively matching each other.

To be more explicit according to the present invention a reservoir with a first collar including a first coding feature matches exclusively a first coding ring with a first counter coding feature such that the first reservoir with the first coding collar may be held in the reservoir holder if and only if the first counter coding feature matches the first coding feature.

A reservoir with a second collar having a second coding feature different than the first coding feature exclusively matches a second coding ring with a second counter coding feature different than first counter coding feature such that the reservoir with the second coding collar may be held in the reservoir holder if and only if the second counter coding feature matches the second coding feature.

For reason of clarity, the term "exclusively" means that only the first coding feature matches the first counter coding feature and the second coding feature matches only the second counter coding feature. That means the reservoir with the second collar and second coding feature does not match to the first counter coding feature and thus the reservoir cannot be held in the reservoir holder.

The fastening element of the coding collar may be a separate element (for example a protrusion, cam, groove) or alternatively it may form the coding feature. In the latter case the fastening element and the coding feature are implemented in one common element.

The reservoir holder is preferably adapted to fully or partly envelop the reservoir and to hold the reservoir in place. In particular, the reservoir holder is adapted to hold the reservoir such that it cannot move relative to a dispensing mechanism housing.

The reservoir holder is preferably fixedly and non-releasably connected to a dose and dispensing mechanism housing. The housing is preferably sleeve-shaped and adapted to accommodate the dispensing mechanism components. In this case, the reservoir holder is preferable fixedly (non-releasably and permanently) connected to the dispensing mechanism. That means the reservoir holder cannot be disconnected from the housing by the user. In particular, the reservoir holder may not be disconnectable from the housing without dedicated tool or without destruction.

Alternatively, the reservoir holder may be integrally formed (as a monolithic part) in a housing which accommodates the dispensing mechanism. That means a (preferably proximal) part of the reservoir holder is a housing adapted to accommodate components of the dispensing mechanism. The dispensing mechanism may include, for example, a dose setting member, a dispensing button or a manual or automatic drive member such as a spring.

The drug delivery device may be a reusable, a disposable, a semi-disposable (also named as semi-reusable) delivery device. The reservoir together with the coding collar are preferably disposed after use or when the reservoir is empty. The dispensing mechanism and the reservoir holder including the coding ring are preferably reusable and can be attached to a new reservoir via a coding collar. The drug delivery device is preferably an injection device, in particular a pen-shaped injection device.

The reservoir may be a container, cartridge (one or two chamber) adapted to contain the drug. The cartridge may be a non-refillable cartridge for single use. The cartridge includes a needle mounting portion where a needle assembly can be releasably attached to. Alternatively, the reservoir may be a prefilled syringe having a needle or cannula that is permanently attached to a first end of a syringe barrel that is sealed at the opposing end by the plunger.

In the present context, the terms "substance", "drug", "medicament" and "medication" are to be understood to include any flowable medical formulation suitable for controlled administration through a means such as, for example, a cannula or a hollow needle, and comprises a liquid, a solution, a gel or a fine suspension containing one or more medical active ingredients. A medicament can be a composition comprising a single active ingredient or a pre-mixed or co-formulated composition with more than one active ingredient present in a single container. Medication includes drugs such as peptides (e.g., insulin, insulin-containing drugs, GLP-1 containing drugs or derived or analogous preparations), proteins and hormones, active ingredients derived from, or harvested by, biological sources, active ingredients based on hormones or genes, nutritional formulations, enzymes and other substances in both solid (suspended) or liquid form but also polysaccharides, vaccines, DNA, RNA, oligonucleotides, antibodies or parts of antibodies but also appropriate basic, auxiliary and carrier substances

The term "distal" is meant to refer to the direction or the end of the drug delivery device carrying an injection needle or an injection cannula, whereas the term "proximal" is meant to refer to the opposite direction or end pointing away from the needle or cannula.

The term "injection device" or "injector" refers to a device that is removed from the injection site after each medication event or drug delivery process, whereas the term "infusion system" refers to a device with a cannula or needle that remains in the skin of the patient for a prolonged period of time, for example, several hours.

In a preferred embodiment the coding collar incudes at least three coding features or/and the ring includes at least two and more preferably three counter coding features. That means at least one of the collar or the ring includes more than one coding feature and the other of the collar and the ring includes a corresponding structure that allows to connect and match the more than one coding feature.

The at least two or at least three coding features or/and the at least two or at least three counter coding features are preferably spaced apart in a circumferential direction such that the collar and the ring can be connected to each other in at least two or three different rotational positions, respectively. That improves the usability as the reservoir can be connected in at least two or at least three different rotational orientation to the coding ring.

Alternatively, or in addition, the collar and/or the ring may include coding features or counter coding features respectively at different positions on the collar or the ring. By way of example, the collar may include a first coding feature on an outer rim and a second coding feature on an inner or outer surface of the collar. Likewise, the coding ring may include a first counter coding feature on a proximal end and a second counter coding feature on inner surface of an opening or on an outer surface of the coding ring.

Preferably, the coding feature is a protrusion extending in the longitudinal axis or a recess adapted to accommodate the protrusion in a connected state and wherein the counter coding feature is the other of the protrusion and the recess.

The protrusion extending along the longitudinal axis provides a safe and reliably coding as an attachment and/or connection of collar to the reservoir holder (via coding ring) can be reliably prevented when the protrusion does not match the recess. As the protrusion extends along the longitudinal axis the coding feature may even support the attachment or coupling process when the reservoir with the collar is about to be attached to the reservoir holder, e.g. when a proximal end portion of the reservoir is inserted into an opening in the reservoir holder and the collar is moved longitudinally to couple the collar to the ring.

Alternatively, the coding feature may be a knob extending along a circumference or it may be thread.

In a preferred embodiment the second connecting portion includes a cam or a L-shaped groove adapted to accommodate the cam. The coding collar preferably includes the other of the cam and the groove to provide a releasable bayonet connection between the coding ring and the coding collar. The bayonet connection provides a safe and reliable connection between the collar with the reservoir and the ring on the reservoir holder. Furthermore, the user can easily fasten or release the bayonet connection which contributes to an enhanced usability.

Preferably, the recess of the coding feature has an elongated form in a circumferential direction. The elongated form allows the coding protrusion to travel within the recess for a rotational movement to fasten and release the bayonet connection.

That means the recess extends in the circumferential direction around the longitudinal axis to allow a twist or rotational movement between the collar and the ring when the coding feature and the counter coding feature match.

In a preferred embodiment the cam or the groove includes a retaining element to hold the cam in an end position within the L-shaped groove when the bayonet connection is fastened. The retaining element is further adapted to increase a breakaway force required to release the bayonet connection.

The breakaway force is the effort or force required to initially move the cam out of its end position within the L-shaped groove and thus to start the release and decoupling of the bayonet connection.

The retaining element ensures that the user needs an increase effort for moving the cam out of the end position within the L-shaped groove. Due to the retaining element an unintentional release of the bayonet connection can be prevented. By way of example, the retaining element may be a bulge, ledge, protrusion, narrowing section or a nose adapted to retain the cam.

In a further preferred embodiment a first coding feature of a first collar matches a first counter coding features of a first ring (first matching pair). A second coding feature of a second collar different from the first coding feature preferably matches a second counter coding feature of a second ring different from the first counter coding feature (second matching pair). The first collar and first ring both have the same colour and the second collar and the second ring both have the same colour which is different from the colour of the first collar and first ring.

The colour coding may support and help the user to find a compatible and matching reservoir with the collar that matches and can be attached to a corresponding coding ring. Additionally or instead of the colour the collar and the ring may include further markers or coding features to show the user which collar with reservoir matches to a specific ring and reservoir holder. By way of example such markers may be a graphic structure a printed or/and three-dimensional patterns or signs, characters, numbers, letters, words or phrases.

The connection between the reservoir holder and the coding ring is preferably a snap-fit connection which is established during a final assembly at the manufacturer site. The snap-fit connection is not intended to be released by the user and may be designed such that the release of the snap-fit connection requires a dedicated tool. The snap-fit connection allows an easy and reliable connection between the coding ring and the reservoir holder.

The counter coding feature of the coding ring may be customized and configured to provide a definite (unique) coding for a particular collar with a reservoir, e.g. for a specific drug or customer. Due to the snap-fit connection the coding ring with the counter coding feature may be easily exchanged to adapted to a specific collar and matching the coding feature.

The snap-fit connection may include, for example, a flexible snap arm on the ring or on the reservoir holder and a recess or opening for the arm on the other of the ring and reservoir holder.

Preferably, a length along the longitudinal axis of the coding ring is less than 25% of a length of the reservoir holder along the longitudinal axis. That means the length of the coding ring is less than a quarter of the length of the reservoir holder. The ring is thus relatively short in comparison to the reservoir holder. That provides a compact design and in particular a compact dimension of the drug delivery device along the longitudinal axis.

Moreover, in a preferred embodiment the maximum outer diameter of the coding ring is equal or less than a maximum outer diameter of the reservoir holder. That means the reservoir holder diameter defines the outer most dimension of the device and the coding ring has the same outer diameter as the reservoir holder or has a smaller diameter as the reservoir holder. That means the coding mechanism does not enlarge the outer dimension of the device.

Preferably, a length along the longitudinal axis of the collar is less than 25% of the length of the reservoir holder. That means the length of the collar is less than a quarter of the length of reservoir holder. The collar is thus compact relative to the length of the reservoir holder or of the reservoir and requires a minimum of space and allows a compact design of the coding mechanism including the collar.

The collar preferably comprises an arm-shaped engaging element adapted to engage the neck portion of the reservoir and preventing a movement of the reservoir relative to the collar along the longitudinal axis. The arm-shaped engaging element provides a simple and reliable fastening to connect the collar to the reservoir such that any movement along the longitudinal axis between the collar and the reservoir is reliably prevented.

The arm-shaped element is preferably integrally formed in the collar, in particular if the collar is an injection molded plastic part the arm-shaped element may be integrally formed and monolithic with the rest of the collar.

Preferably, a proximal portion of the reservoir holder is fixedly, non-releasably and permanently connected to a distal end portion of a housing of the dispensing mechanism. The reservoir holder is not intended to be disconnected and/or removed from the housing by the user. The reservoir holder is preferably connected to the housing by the manufacturer, for example, by a non-releasable snap-fit connection.

In this embodiment, the reservoir holder has preferably approximatively the length of the reservoir. That means the reservoir holder completely envelops the reservoir and is adapted to hold the reservoir in place relative to the housing which accommodates the dose and dispensing mechanism of the drug delivery device. The reservoir holder is preferably sleeve-shaped.

In an alternative embodiment the reservoir holder may not be a separate part but may be integrally formed and monolithic with the housing. In this alternative embodiment the reservoir holder extends along an entire length of the dispensing mechanism along the longitudinal axis and the reservoir holder is adapted to accommodate the reservoir in a distal portion and is further adapted to envelop the dispensing mechanism in a proximal portion of the reservoir holder. The reservoir holder is in this embodiment works thus also as a housing for the dose and dispensing mechanism. The reservoir holder is preferably the outer most part of the drug delivery device.

Therefore, the function of holding the reservoir in place and housing the dose and dispensing mechanism is implemented in one single part which reduces number of delivery device components and the complexity of the device.

The invention further relates to a method for connecting a reservoir assembly to a dose and dispensing mechanism of a drug delivery device. The method comprises the steps of
a) Fastening, preferably in a final assembly step, non-releasably, a neck portion of a reservoir to a coding collar;
b) Connecting, preferably in a final assembly step, by a non-releasably connection, a coding ring to a distal end of a reservoir holder via a first connecting portion of the coding ring;
c) Releasably connecting by a user, via a second connecting portion of the coding ring, the coding ring to the coding collar;
d) Matching, during the connecting, a coding feature of the collar to a counter coding feature of the ring.

### BRIEF DESCRIPTION OF THE DRAWINGS

The subject matter of the invention will be explained in more detail in the following text with reference to preferred exemplary embodiments which are illustrated in the attached drawings, in which:
- Fig.1: depicts a perspective view of an injection pen with a reservoir assembly according to the invention;
- Fig.2: depicts the injection pen with the reservoir assembly disconnected from the coding ring;
- Fig.3: depicts a perspective view of a coding collar;
- Fig. 4: depicts a sectional view of the injection pen with an attached reservoir assembly;
- Fig. 5: depicts a second embodiment of an injection pen with a reservoir assembly;
- Fig. 6: depicts a third embodiment of an injection pen with a reservoir assembly;
- Fig. 7: depicts a sectional view of the injection pen of the third embodiment.

The reference symbols used in the drawings, and their primary meanings, are listed in summary form in the list of designations. In principle, identical parts are provided with the same reference symbols in the figures.

### DETAILED DESCRIPTION OF PREFERRED EMBODIMENTS

In the present description the term "distal" refers to the side where the injection needle is located. This is on the left-hand side in the figures. The term "proximal" refers to the opposite side or rear end of the device and is on the right-hand side in the figures.

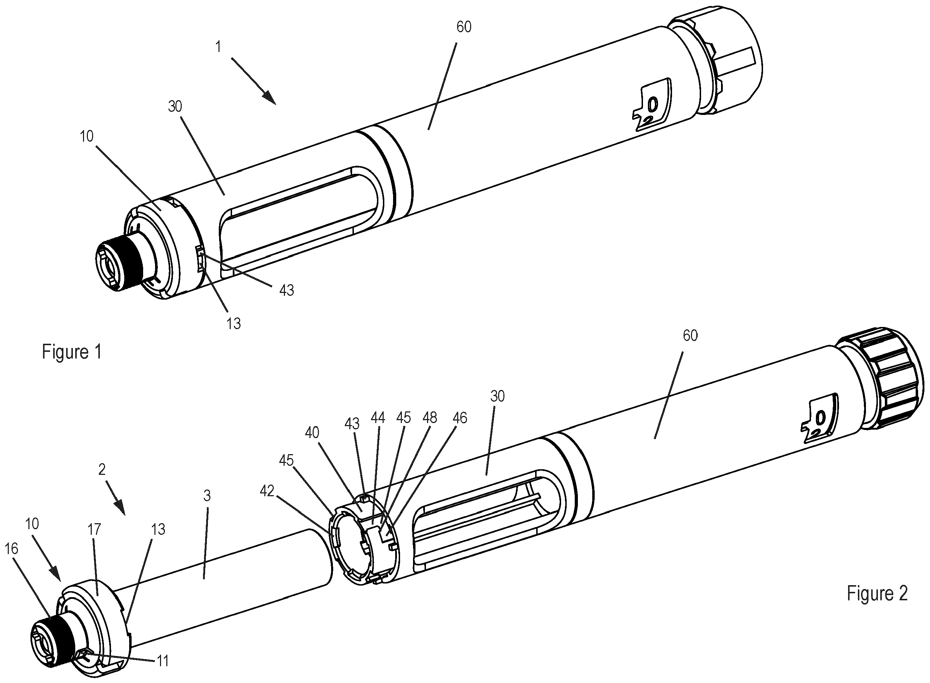

Figure 1 depicts a drug delivery device in form of a reusable injection pen 1 with coding features according to the invention. Figure 2 depicts a perspective view of the injection pen 1 with a coding collar and a cartridge 3 decoupled and separated from a reservoir holder 30 and a housing 60 of the injection pen. The reservoir assembly 2 includes the coding collar 10 non-releasably connected to a neck portion of the cartridge 3 and a reservoir holder 30 and a coding ring 40 fixedly connected to the reservoir holder 30. The injection pen 1 comprises the reservoir assembly 2, the housing 60 and a dose and dispensing mechanism accommodated inside the housing 60. The collar 10 together with the cartridge can be attached to and detached from the reservoir holder 30 by the user to exchange a cartridge.

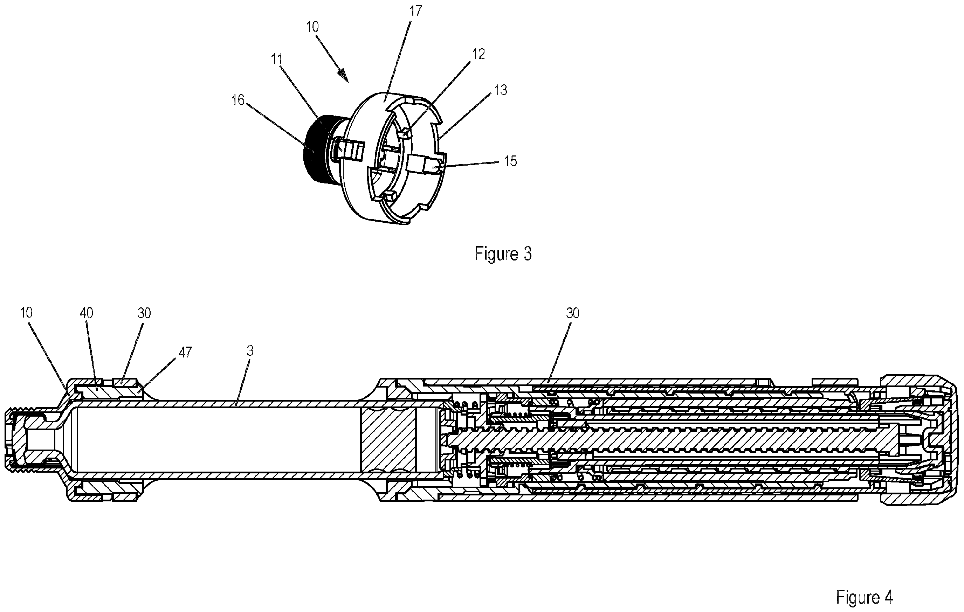

As best shown in figure 2 the coding collar 10 is connected to a neck portion of the cartridge 3. For that purpose, the collar 10 comprises two oppositely arranged snap arms 11 (figure 2 and 3) which engage and clamp the cartridge neck portion. The cartridge 3 is therefore fixedly and non-releasably connected to the collar 10. The collar 10 is mounted to the cartridge at the manufacturer site and is not intended to be removed from the cartridge 3 by the user.

The snap arms 11 are located between a distal needle mounting portion 16 which is adapted to be releasably attached to a needle assembly with an injection needle and a sleeve or ring-shaped proximal portion 17 with a larger diameter than the needle mounting portion 16, see figures 2 and figure 3.

Figure 3 depicts a perspective view of the coding collar 10 alone without cartridge. As it can be seen in figure 3 the proximal portion 17 of the collar has an opening adapted to accommodate the neck portion of the cartridge 3. Inside the opening the collar 10 includes two oppositely arranged cams 15 adapted to be inserted into the L-shaped grooves 45 in the coding ring 40 to fasten a bayonet connection between the collar 10 and the ring 40. On an inside of the opening the collar 10 further includes four coding protrusions 12 evenly distributed along the circumference. In an end rim (proximally faced end surface) the collar 10 comprises four coding recesses 13 which have an elongated form in the circumferential direction and which are evenly distributed along the circumference.

With the above-described coding collar 10 and the coding ring 40 the reservoir assembly 2 provides three pairs of coding and counter coding features. A first coding is provided by the cams 15 which are designed such that the cams exclusively match the L-shaped grooves 45 of the ring. The grooves 45 thus acts as counter coding features. A second coding is provided by the coding protrusions 12 on the inside of the collar 10. The protrusions 12 exclusively match counter recesses 42 in the ring (figure 2). The collar coding recesses 13 on the proximal end of the collar provide a third coding as counter protrusions 43 of the ring are designed to exclusively match the recesses 13 of the collar 10. All recesses 13, 42 have an elongated form and are designed such that the protrusions 12, 43 can travel within the recesses during relative rotation between the collar 10 and the ring 40 when the bayonet connection is fastened or released.

The coding ring 40 comprises a first connection portion including two oppositely positioned snap arms 47 for a snap fit connection with a distal end portion of the reservoir holder 30 as shown in figure 4. Figure 4 depicts a sectional view where the cut for the section runs along the longitudinal axis of the injection pen. The ring 40 is thus immovably held to the reservoir holder 30 and is not intended to be decoupled and separated from the reservoir holder by the user after assembly. The ring 40 along with the reservoir holder 30 provides an opening to accommodate the cartridge 3 in an attached state as shown in figure 1 and figure 4.

The coding ring 40 further includes a second connection portion on a distal end including the L-shaped grooves 45 (figure 2) adapted to accommodate the cams 15 of the collar for the bayonet connection between the ring 40 and the collar 10. The ring 40 includes in total four L-shaped grooves 45 along the circumference spaced apart from each other in an angle of 90°. Therefore, the collar 10 can be connected to the ring 40 in four different rotational orientations. As shown in figure 2 each L-shaped groove 45 comprises an axial groove section 44 and a radial groove section 46. At an edge between the axial and radial groove section 44, 46 a retaining element in form of a retaining nose 48 is located. When the cam 15 enters or leaves the radial section and the cam 15 has the pass the narrow section formed by the nose 48 which requires an extra force. That means the retaining nose 48 holds the cam 15 in its end position such that an unintentional release of the bayonet connection can be prevented.

Next to each L-shaped groove 45 and thus in total four coding protrusions 43 are positioned on an outside of the ring. The protrusions 43 exclusively match the recesses 13 in the collar 10 as described above. Additionally, the ring 40 comprises on an inside of a distal end rim four coding recesses 42 which are 90° spaced apart from each other along the circumference. The coding recesses 42 are designed to match the coding protrusion 12 of the collar 10.

A length along the longitudinal axis of the coding ring 40 is less than a quarter of a length of the reservoir holder 30 and a length along the longitudinal axis of the collar 10 is less than a quarter of the length of the reservoir holder 30. Further, a maximum outer diameter of the coding ring 40 is equal than a maximum outer diameter of the reservoir holder 30. The coding provides thus a compact design.

The coding collar 10 which matches a coding ring 40 (matching combination) may have the same colour to support the user to connect a compatible and correct collar and cartridge with a corresponding ring of a reservoir holder.

In order to replace a cartridge 3, the user grabs the collar 10 and rotates the collar 10 relative to the reservoir holder 30 and thus relative to the ring 40. That causes the cams 15 to move out of their end position within the L-shaped grooves 45 in the ring 40. As the cams 15 have to pass the narrow sections formed by the retaining noses 48 a breakaway force is required to release the bayonet connection. When the cams 15 are in the axial groove sections the user axially pulls the collar 10 together with the cartridge 3 away from the reservoir holder. The user can then remove the collar with cartridge and can discard it.

A new cartridge 3 with a new collar 10 intended for use with the corresponding injection pen (and coding ring 40) has to be chosen. To attach the new cartridge to the reservoir holder the user inserts a proximal end of the cartridge 3 into the opening of the ring 40 and the reservoir holder 30. If the coding of the collar 10 matches the counter coding of ring the cams 15 of the collar 10 can be inserted into the L-shaped grooves 45 and the collar coding protrusions 12 match the coding recesses 42 of the ring and the coding recesses 13 of the collar match the coding protrusion 43 of the ring. In order to completely attach the cartridge 3 and close and fasten the bayonet connection the user rotates the collar 10 relative to the ring 40 and thereby moves the cams 15 inside the radial groove sections 46. Each cam 15 has to pass the retaining nose 48 and is moved in its end position and held in place by the retaining nose 48.

If the user has not combined the correct cartridge the protrusions 12, 43 do not match the corresponding recesses 42, 13 due to their size or/and the position or/and the user cannot insert the cams 15 into the L-shaped grooves 45. In this case the bayonet connection cannot be established. That means the cartridge 3 cannot be attached to the injection pen housing via coding collar 10 and coding ring 40.

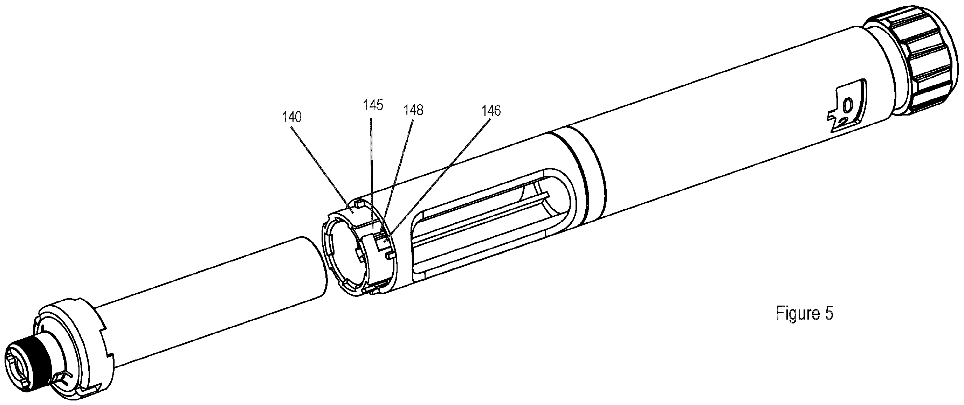

Figure 5 depicts a perspective view of a second embodiment of an injection pen with a reservoir assembly according to the present invention. In contrast to the first embodiment in the second embodiment each L-shaped groove 145 in the coding ring 140 includes a retaining element in form of radially extending bulge 148 on the bottom of the radial groove section 146. That means if the cam 15 of the collar 10 is in its end position in the radial groove section 146 the cam 15 is held in place and thus the bayonet connection remains closed. When the user intends to release the collar 10 from the ring 140 a breakaway force is required to bring the cam over the retaining bulge 148. An unintentional release of the bayonet connection can thus be prevented by the retaining bulge 148.

All other features of the second embodiment correspond to the features described with respect to the first embodiment according to figures 1 to 4.

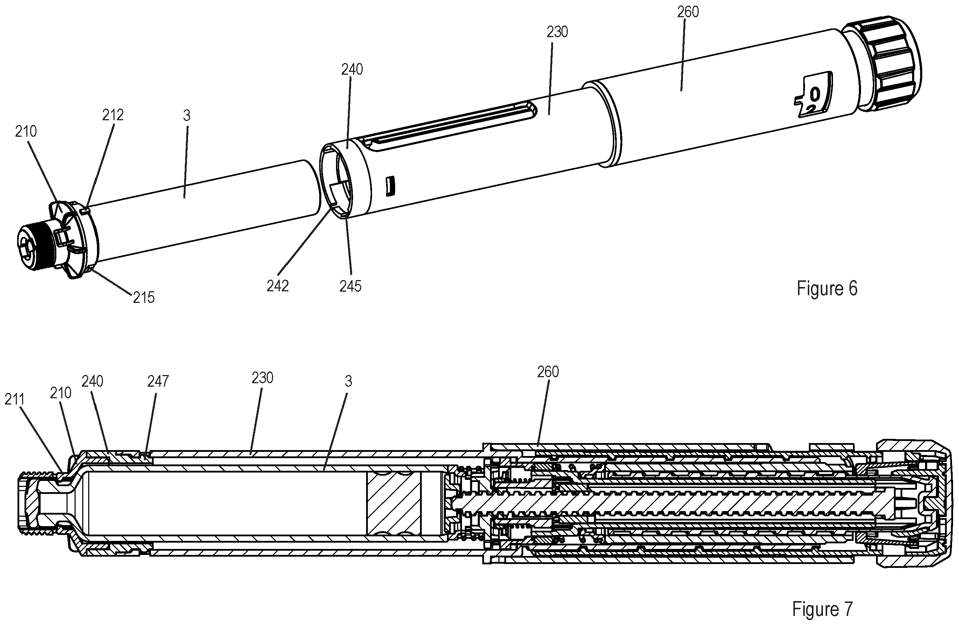

Figures 6 and 7 depict a third embodiment of the reservoir assembly according to the present invention.

The coding collar 210 of the third embodiment comprises in its proximal portion coding features in form of two oppositely arranged and radially extending protrusions 212 and to oppositely arranged cams 215. The position and the dimensions of the protrusions 212 and cam 215 form the coding feature. Alternatively, the collar may include three or even four protrusion and cams extending radially and evenly distributed around the circumference.

The cams 215 are adapted to engage L-shaped grooves 245 in the coding ring 240 to establish a bayonet connection as described above.

The ring 240 of the third embodiment is fixedly connected to a housing 260 which accommodates the dose and dispensing mechanism. The ring is non-releasably connected to the reservoir holder 230 by two oppositely arranged snap arms 247 which engage corresponding openings in the reservoir holder as best shown in figure 7. Figure 7 depicts a sectional view where the cut for the sectional view runs along the longitudinal axis of the injection pen.

Furthermore, the ring 240 includes two axial recesses 242 for the protrusions 212 where each axial recess 242 leads to an L-shaped groove 245. In an attached state the protrusions 212 are located within the L-shaped grooves 245 and can travel therein during a rotational fastening movement when the collar 210 is rotated relative to the ring 240. The other features of the collar 210 and ring 240 correspond to the first and second embodiment as described above.

While the invention has been described in detail in the drawings and foregoing description, such description is to be considered illustrative or exemplary and not restrictive. Variations to the disclosed embodiments can be understood and effected by those skilled in the art and practising the claimed invention, from a study of the drawings, the disclosure, and the appended claims. In the claims, the word "comprising" does not exclude other elements or steps, and the indefinite article "a" or "an" does not exclude a plurality. The mere fact that certain elements or steps are recited in distinct claims does not indicate that a combination of these elements or steps cannot be used to advantage, specifically, in addition to the actual claim dependency, any further meaningful claim combination shall be considered disclosed.

**LIST OF DESIGNATIONS**

| | | |
|---|---|---|
| 1 | injection pen | 140 coding ring |
| 2 | reservoir assembly | 145 L-shaped groove |
| 3 | cartridge | 148 retaining bulge |
| | | 146 radial groove section |
| 10 | coding collar | 210 coding collar |
| 11 | snap arm | 212 protrusion |
| 12 | coding protrusion | 215 cam |
| 13 | coding recess | |
| 15 | cam | 230 reservoir holder |
| 16 | needle mounting portion | |
| 17 | proximal portion | 240 coding ring |
| | | 242 axial recess |
| 30 | reservoir holder | 245 L-shaped groove |
| | | 247 snap arms |
| 40 | coding ring | 260 housing |
| 41 | | |
| 42 | coding recess | |
| 43 | coding protrusion | |
| 44 | axial groove section | |
| 45 | L-shaped groove | |
| 46 | radial groove section | |
| 47 | snap arm | |
| 48 | retaining nose | |
| 60 | housing | |

## Claims

1. Reservoir assembly (2) for a drug delivery device (1) for dispensing a liquid drug from a reservoir (3), the reservoir assembly (2) comprising
- a reservoir holder (30) defining a longitudinal axis (L) with a distal and proximal end and adapted to be connected to a dispensing mechanism of the delivery device (1) and wherein the reservoir holder (30) is adapted to hold the reservoir (3) in place relative to the dispensing mechanism;
- a coding collar (10) comprising a fastening element (11) for connecting the collar (10) non-releasably to a neck portion of the reservoir (3), the collar (10) comprising a coding feature (12),
**characterized in that** the reservoir assembly (2) further comprises a coding ring (40) comprising
- a first connecting portion to attach non-releasably the coding ring (40) to the distal end of the reservoir holder (30);
- a second connecting portion to releasably couple the coding ring (40) to the collar (10) with the reservoir (3), wherein the second connection portion includes a counter coding feature (42) such that the reservoir (2) may be held in the reservoir holder (30) only if the counter coding feature (42) matches the coding feature (12).

2. Reservoir assembly (2) according to claim 1, wherein the collar (10) includes at least three coding features (12) or the ring (40) includes at least three counter coding features (42), wherein the at least three coding features (12) or the at least three counter coding features (42) are respectively identical and spaced apart in a circumferential direction such that the collar (10) and the ring (40) can be connected to each other in at least three different rotational positions.

3. Reservoir assembly (2) according to claim 1 or 2, wherein the coding feature (12) is a protrusion extending in the longitudinal axis or a recess adapted to accommodate the protrusion in a connected state and wherein the counter coding feature (42) is the other of the protrusion and the recess.

4. Reservoir assembly (2) according to any of claims 1 to 3, wherein the second connecting portion of the ring (40) includes a cam (15) or a L-shaped groove (45) adapted to accommodate the cam and the collar (10) includes the other of the cam (15) and the groove (45) to provide a releasable bayonet connection between the collar (10) and the ring (40).

5. Reservoir assembly (2) according to claim 3 and 4, wherein the recess (13) has an elongated form in a circumferential direction such that the protrusion (12) can travel within the recess (13) for a rotational movement to establish the bayonet connection.

6. Reservoir assembly (2) according to claim 4 or 5, wherein the cam (15) or the groove (45) includes a retaining element (48) adapted to increase a breakaway force required to release the bayonet connection.

7. Reservoir assembly (2) according to any of claims 1 to 6, wherein a first coding feature (12) of a first collar (10) matches a first counter coding feature (42) of a first ring (40) and a second coding feature of a second collar different from the first coding feature matches a second counter coding feature of a second ring different from the first counter coding feature, wherein the first collar and first ring both have a same color and the second collar and the second ring both have a same color which is different from the color of the first collar and ring.

8. Reservoir assembly (2) according to any of claims 1 to 6, wherein the connection between the reservoir holder (30) and the coding ring (40) is a snap-fit connection.

9. Reservoir assembly (2) according to any of claims 1 to 8, wherein a length along the longitudinal axis of the coding ring (40) is less than 25% of a length of the reservoir holder (30).

10. Reservoir assembly (2) according to any of claims 1 to 9, wherein a maximum outer diameter of the coding ring (40) is equal or less than a maximum outer diameter of the reservoir holder (30).

11. Reservoir assembly (2) according to any of claims 1 to 10, wherein a length along the longitudinal axis of the collar (10) is less than 25% of the length of the reservoir holder (30).

12. Reservoir assembly (2) according to any of claims 1 to 11, wherein the fastening element (11) of the collar (10) is a snap arm adapted to engage the neck portion and preventing a movement of the reservoir (3) relative to the collar (10) along the longitudinal axis.

13. Reservoir assembly (2) according to any of claims 1 to 12, wherein a proximal portion of the reservoir holder (30) is permanently connected to a distal end portion of a housing of the dispensing mechanism.

14. Reservoir assembly (2) according to any of claims 1 to 12, wherein the reservoir holder (30) extends along an entire length of the dispensing mechanism along the longitudinal axis and wherein the reservoir holder (30) is adapted to envelop the dispensing mechanism in a proximal portion of the reservoir holder (30).

15. Method for connecting a reservoir assembly according to any previous claim to a dispensing mechanism of a drug delivery device (1), the method comprising the steps of
a) Fastening, non-releasably, a neck portion of a reservoir (3) to a coding collar (10);
b) Connecting, by a non-releasably connection, a coding ring (40) to a distal end of a reservoir holder (30) via a first connecting portion of the coding ring (40);
c) Releasably connecting, via a second connecting portion of the coding ring (40), the coding ring (40) to the coding collar (10);
d) Matching, during the connecting, a coding feature (12) of the collar to a counter coding feature (42) of the ring.
